(19) Europäisches Patentamt
European Patent Office
Office européen des brevets

(11) EP 3 714 873 A1

# (12) EUROPÄISCHE PATENTANMELDUNG

(43) Veröffentlichungstag:
**30.09.2020 Patentblatt 2020/40**

(21) Anmeldenummer: **19166142.0**

(22) Anmeldetag: **29.03.2019**

(51) Int Cl.:
***A61K 9/00*** *(2006.01)* ***A61K 47/12*** *(2006.01)*
***A61K 47/20*** *(2006.01)* ***A61K 47/10*** *(2017.01)*
***A61K 31/465*** *(2006.01)* ***A24D 1/00*** *(2020.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **VitaSalts AG**
**9496 Balzers (LI)**

(72) Erfinder: **Endler, Stephan**
**8232 Merishausen (CH)**

(74) Vertreter: **Michalski Hüttermann & Partner**
**Patentanwälte mbB**
**Speditionstraße 21**
**40221 Düsseldorf (DE)**

## (54) BASISFLÜSSIGKEIT FÜR VERDAMPFUNGSZUSAMMENSETZUNGEN ZUM INHALIEREN

(57) Die vorliegende Erfindung betrifft Basisflüssigkeiten für Verdampfungszusammensetzungen zum Inhalieren. Die Basisflüssigkeit für Verdampfungszusammensetzungen zum Inhalieren bestehend zumindest aus:
- einem Hilfsstoff,
- und als Rest einer Mischung bestehend aus Propylenglykol in einem Bereich von größer oder gleich 0 gew.-% bis kleiner oder gleich 100 gew.-%, Glycerin in einem Bereich von größer oder gleich 0 gew.-% bis kleiner oder gleich 100 gew.-% und Wasser in einem Bereich von größer oder gleich 0 gew.-% bis kleiner oder gleich 50 gew.-%,
wobei der Hilfsstoff einen Puffer bildet und zumindest einer ersten und einer zweiten Puffersubstanz eines Puffersystems besteht.

EP 3 714 873 A1

## Beschreibung

Die vorliegende Erfindung betrifft Basisflüssigkeiten für Verdampfungszusammensetzungen zum Inhalieren.

Verdampfungszusammensetzungen zum Inhalieren sind an sich bekannt und finden insbesondere im Bereich elektronischer Zigaretten Anwendung. Dabei werden Flüssigkeiten mit elektronischen Zigaretten verdampft. Der Dampf wird von einem Konsumenten üblicherweise durch den Mund in die Lungen inhaliert. Von besonderem Interesse sind dabei nikotinhaltige und aromatisierte Verdampfungszusammensetzungen, wobei das Nikotin durch Inhalation des Dampfes in den Blutkreislauf gelangt um seine Wirkung zu entfalten.

Verdampfungszusammensetzungen werden zum Teil kommerziell als verdampfungsfertige Zusammensetzung hergestellt. Einige Konsumenten stellen verdampfungsfertige Verdampfungszusammensetzungen jedoch auch privat selbst her, um individuelle Aromamischungen und Aromaintensitäten sowie auch individuelle Nikotinstärken zu erhalten. Sowohl in der kommerziellen als auch in der privaten Herstellung werden Basisflüssigkeiten für Verdampfungszusammensetzungen benötigt. Sie werden zumeist in einem separaten Schritt in großen Mengen hergestellt oder eingekauft.

Basisflüssigkeiten für Verdampfungszusammensetzungen zum Inhalieren sind an sich bekannt.

Basisflüssigkeiten für Verdampfungszusammensetzungen zum Inhalieren können jedoch noch Verbesserungspotential bieten. Verbesserungspotential kann sich dabei insbesondere in dem Einfluss der Basisflüssigkeiten auf die Eigenschaften von verdampfungsfertigen Verdampfungszusammensetzungen zum Inhalieren bieten. Insbesondere kann der Einfluss auf die Lagerstabilität der Verdampfungszusammensetzung, auf den Geschmack der Verdampfungszusammensetzung, die Resorbierbarkeit von beispielsweise beinhaltetem Nikotin einer Verdampfungszusammensetzung oder den Throat-Hit einer Verdampfungszusammensetzung Verbesserungspotential bieten.

Es ist daher die Aufgabe der vorliegenden Erfindung, eine verbesserte Basisflüssigkeit für Verdampfungszusammensetzungen zum Inhalieren bereitzustellen.

Gelöst wird diese Aufgabe durch Basisflüssigkeiten für Verdampfungszusammensetzungen zum Inhalieren gemäß Anspruch 1. Bevorzugte Ausgestaltungen der Erfindung sind in den Unteransprüchen und in der Beschreibung angegeben, wobei weitere in den Unteransprüchen oder in der Beschreibung beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Mit der Erfindung wird eine Basisflüssigkeit für Verdampfungszusammensetzungen zum Inhalieren vorgeschlagen.

Die Basisflüssigkeit für Verdampfungszusammensetzungen zum Inhalieren, besteht dabei zumindest aus:

- einem Hilfsstoff,
- und als Rest einer Mischung bestehend aus Propylenglykol in einem Bereich von größer oder gleich 0 gew.-% bis kleiner oder gleich 100 gew.-%, Glycerin in einem Bereich von größer oder gleich 0 gew.-% bis kleiner oder gleich 100 gew.-% und Wasser in einem Bereich von größer oder gleich 0 gew.-% bis kleiner oder gleich 50 gew.-%.

Dabei ist vorgesehen, dass der Hilfsstoff einen Puffer bildet und zumindest aus einer ersten und einer zweiten Puffersubstanz eines Puffersystems besteht.

Es konnte in überraschender Weise gezeigt werden, dass die erfindungsgemäßen Basisflüssigkeiten für Verdampfungszusammensetzungen zum Inhalieren sich besonders gut als Basisflüssigkeit zur Mischung mit Aromen und/oder Nikotin eignen. Dabei konnte mit einer erfindungsgemäßen Basisflüssigkeit eine verbesserte Lagerstabilität von Aromen erreicht werden. Zudem konnte in überraschender Weise gezeigt werden, dass mit einer erfindungsgemäßen Basisflüssigkeit die Resorbierbarkeit und Resorptionsgeschwindigkeit von Nikotin beim Inhalieren einer nikotinhaltigen Verdampfungszusammensetzung einstellbar ist B. Koszowski, L. C. Viray, S. B. Stanfill, J. G. Liskob, Z. R. Rosenberry, J. L. Potts, W. B. Pickworth, Pharmacol Biochem Behav. 2015, 136, 1-6) oder R. V Fant, J. E Henningfield, R. A Nelson,W. B Pickworth , Tobacco Control, 1999; 8,:387-392. W. B. Pickworth, Z. R. Rosenberry, W. Gold, B. Koszowski, J Addict Res Ther, 2014, 5(3), 1), sowie der Geschmackseinfluss des Nikotins und der Throat-Hit nikotinhaltiger Verdampfungszusammensetzungen zum Inhalieren. Insbesondere konnte gezeigt werden, dass sich diese Eigenschaften im Wesentlichen unabhängig von der exakten Menge der in üblichen Verhältnissen zugesetzten Stoffe wie Aromen oder Nikotin erreichen lassen können.

Unter Nikotin ist im Rahmen der vorliegenden Erfindung das natürlich in den Blättern der Tabakpflanze vorkommende Alkaloid zu verstehen, das auch unter den Namen (S)-(-)-3-(1-Methyl-pyrrolidin-2-yl)pyridin, (S)-(-)-1-Methyl-2-(3-pyridyl)pyrrolidin, oder L-3-Pyridyl-N-methylpyrrolidin bekannt ist.

Unter Aroma ist im Sinne der vorliegenden Erfindung eine Verbindung oder eine Mischung von Verbindungen zu verstehen, die einen vorzugsweise angenehmen, charakteristischen Geschmack oder Geruch aufweist.

Unter Basisflüssigkeit ist im Sinne der vorliegenden Erfindung eine Flüssigkeit zu verstehen, die verdampfbar ist und in der der Hilfsstoff und optional Aroma und Nikotin im Wesentlichen lösbar sind.

Unter Resorbierbarkeit ist im Sinne der vorliegenden Erfindung insbesondere die Menge resorbierten

Nikotins im Verhältnis zur Menge inhalierten Nikotins zu verstehen.

Unter Throat-Hit ist im Sinne der vorliegenden Erfindung das kratzende Gefühl im Rachen beim Inhalieren von Dampf einer Verdampfungszusammensetzungen zu verstehen.

Ohne an eine Theorie gebunden zu sein wird davon ausgegangen, dass durch den erfindungsgemäßen Hilfsstoff die Basisflüssigkeit als Puffer für etwaige weitere Zusatzstoffe dient. Dadurch können derartige Zusatzstoffe, wie beispielsweise Aromen oder Nikotin stabilisiert werden.

Eine vorbeschriebe Basisflüssigkeit dient somit insbesondere zur Einstellung der Resorbierbarkeit und Resorptionsgeschwindigkeit von etwaigem Nikotin beim Inhalieren eines Dampfes einer nikotinhaltigen Verdampfungszusammensetzung auf Basis einer erfindungsgemäßen Basisflüssigkeit und einer verbesserten Verdampfbarkeit des Nikotins, sowie insbesondere einer verbesserten Lagerstabilität von Verdampfungszusammensetzung auf Basis einer erfindungsgemäßen Basisflüssigkeit.

Durch die Wirkung als Puffer ist die Basisflüssigkeit dabei besonders als Basis zur Herstellung für Verdampfungszusammensetzungen im privaten Bereich geeignet, da die positiven Eigenschaften über einen großen Bereich unabhängig von der Menge an zugesetztem Nikotin oder Aroma erreicht werden können.

Die Basisflüssigkeit für Verdampfungszusammensetzungen zum Inhalieren kann durch Zusammengeben der einzelnen Bestandteile unter Rühren und optional unter Erwärmung mit üblichen Verfahren hergestellt werden.

Eine Verdampfungszusammensetzung zum Inhalieren auf Basis der erfindungsgemäßen Basisflüssigkeit kann beispielsweise mit einer üblichen elektronischen Zigarette verdampft werden und der entstandene Dampf kann vom Konsumenten auf bekannte Weise inhaliert werden.

In einer bevorzugten Ausgestaltung der Erfindung kann vorgesehen sein, dass die zweite Puffersubstanz eine korrespondierende Base oder eine korrespondierende Säure der ersten Puffersubstanz ist.

Dadurch kann vorteilhafter Weise erreicht werden, dass der Hilfsstoff einen stabilen Puffer bildet.

Unter einer korrespondierenden Base oder Säure ist im Sinne der vorliegenden Erfindung die Base oder Säure zu verstehen die durch Deprotonierung oder Protonierung der entsprechenden Säure oder Base entsteht. Korrespondierende Basen oder Säuren sind auch unter dem Namen konjugierte Basen oder Säuren bekannt.

Dabei kann insbesondere vorgesehen sein, dass die erste Puffersubstanz eine Säure ist und die zweite Puffersubstanz eine korrespondierende Base dieser Säure ist. Alternativ kann insbesondere vorgesehen sein, dass die erste Puffersubstanz eine Base ist und die zweite Puffersubstanz eine korrespondierende Säure dieser Base ist.

In einer bevorzugten Ausgestaltung der Erfindung kann vorgesehen sein, dass der Hilfsstoff ein molares Verhältnis von der ersten Puffersubstanz zu der zweiten Puffersubstanz aufweist in einem Bereich von größer oder gleich 0,25:1 bis kleiner oder gleich 4:1, vorzugsweise in einem Bereich von größer oder gleich 0,5:1 bis kleiner oder gleich 2:1, besonders bevorzugt in einem Bereich von größer oder gleich 0,9:1 bis kleiner oder gleich 1,1:1

Dadurch kann vorteilhafter Weise erreicht werden, dass die Basisflüssigkeit eine große Pufferkapazität aufweist.

In einer bevorzugten Ausgestaltung der Erfindung kann vorgesehen sein, dass die zweite Puffersubstanz ein Salz ist, vorzugsweise ausgewählt aus der Gruppe bestehend aus Ammonium-, Lithium-, Natrium-, Kalium-, Magnesium- und/oder Calcium-Salzen oder aus der Gruppe bestehend aus Chlorid-, Bromid-, Iodid-, Sulfat-, Phosphat-, Nitrat-, Carbonat- und Thiocyanat-Salzen.

Dadurch kann vorteilhafter Weise erreicht werden, dass der Puffer besonders einfach gebildet werden kann.

In einer bevorzugten Ausgestaltung der Erfindung kann vorgesehen sein, dass die zweite Puffersubstanz ein Ammonium-Salz und/oder Natrium-Salz einer korrespondierenden Base der ersten Puffersubstanz ist.

In einer alternativen Ausgestaltung der Erfindung kann vorgesehen sein, dass die zweite Puffersubstanz ein Chlorid-Salz oder Sulfat-Salz einer korrespondierenden Säure der ersten Puffersubstanz ist.

In einer Ausgestaltung der Erfindung kann vorgesehen sein, dass die erste und die zweite Puffersubstanz separat voneinander in die Basisflüssigkeit gegeben werden.

In einer alternativen Ausgestaltung der Erfindung kann vorgesehen sein, dass die zweite Puffersubstanz aus der ersten Puffersubstanz in der Basisflüssigkeit hergestellt wird.

Dabei kann vorgesehen sein, dass die zweite Puffersubstanz aus der ersten Puffersubstanz direkt in der Basisflüssigkeit hergestellt wird. Insbesondere kann vorgesehen sein, dass die erste Puffersubstanz eine Säure ist und zur Basisflüssigkeit hinzugegeben wird, wobei anschließend eine starke Base zu der Basisflüssigkeit gegeben wird, beispielsweise Natriumhydroxid, wobei die zweite Puffersubstanz in der Basisflüssigkeit entsteht. Alternativ kann vorgesehen sein, dass die erste Puffersubstanz eine Base ist und zur Basisflüssigkeit hinzugegeben wird, wobei anschließend eine starke Säure zur der Basisflüssigkeit gegeben wird, beispielsweise Salzsäure, wobei die zweite Puffersubstanz in der Basisflüssigkeit entsteht.

In einer bevorzugten Ausgestaltung der Erfindung kann vorgesehen sein, dass die Basisflüssigkeit den zumindest einen Hilfsstoff in einem Verhältnis bezogen auf die Basisflüssigkeit aufweist in einem Bereich

von größer oder gleich 0,2 gew.-% bis kleiner oder gleich 20 gew.-%, vorzugsweise von größer oder gleich 0,5 gew.-% bis kleiner oder gleich 10 gew.-%, besonders bevorzugt von größer oder gleich 1 gew.-% bis kleiner oder gleich 5 gew.-%.

Dadurch kann vorteilhafter Weise erreicht werden, dass die Basisflüssigkeit eine ausreichende Pufferkapazität aufweist um Aromen oder Nikotin in üblichen Mengen abzupuffern, ohne Verdampfungseigenschaften oder den Geschmack einer Verdampfungszusammensetzung zum Inhalieren auf Basis der erfindungsgemäßen Basisflüssigkeit zu stark zu beeinflussen.

In einer bevorzugten Ausgestaltung der Erfindung kann vorgesehen sein, dass die erste Puffersubstanz einen ersten pKs-Wert aufweist in einem Bereich von größer oder gleich -5 bis kleiner oder gleich 10, vorzugsweise größer oder gleich -2 bis kleiner oder gleich 5, besonders bevorzugt größer oder gleich -2 bis kleiner oder gleich 4.

Unter einem pKs-Wert ist im Sinne der vorliegenden Erfindung der negative dekadische Logarithmus der Säurekonstanten Ks zu verstehen, die die Lage des Gleichgewichts der Reaktion der Puffersubstanz mit Wasser unter Protolyse angibt. Für eine Substanz HA und die Gleichgewichtsreaktion von HA durch Protolyse mit Wasser zu $A^-$ und $H_3O^+$ ist der pKs-Wert definiert als:

$$pKs = -\lg\left(\frac{c(H_3O^+) * c(A^-)}{c(HA)} * 1\frac{L}{mol}\right)$$

wobei $c(H_3O^+)$ die Konzentration von $H_3O^+$, $c(A^-)$ die Konzentration der deprotonierten Substanz, und c(HA) die Konzentration der nicht deprotonierten Substanz im Gleichgewicht in mol/L ist.

Dadurch kann vorteilhafter Weise erreicht werden, dass die Basisflüssigkeit einen Puffer mit einem pH-Wert in einem vorteilhaften Bereich aufweist.

In einer bevorzugten Ausgestaltung der Erfindung kann vorgesehen sein, dass die erste Puffersubstanz einen zweiten und optional dritten pKs-Wert aufweist in einem Bereich von größer oder gleich 0 bis kleiner oder gleich 10, vorzugsweise größer oder gleich 2 bis kleiner oder gleich 8, besonders bevorzugt größer oder gleich 3,5 bis kleiner oder gleich 6,5.

Dadurch kann vorteilhafter Weise erreicht werden, dass die Basisflüssigkeit neben einem ersten Pufferbereich auch einen zweiten Pufferbereich aufweist. Dadurch kann gegebenenfalls eine noch größere Menge an Zusatzstoffen wie Nikotin oder Aromen abgepuffert werden.

Unter einem zweiten pKs-Wert ist im Sinne der vorliegenden Erfindung der negative dekadische Logarithmus der zweiten Säurekonstanten Ks zu verstehen, die die Lage des Gleichgewichts der weiteren Reaktion der bereits deprotonierten Puffersubstanz mit Wasser unter Protolyse angibt. Unter einem dritten pKs-Wert ist im Sinne der vorliegenden Erfindung entsprechend der negative dekadische Logarithmus der dritten Säurekonstanten Ks zu verstehen, die die Lage des Gleichgewichts der weiteren Reaktion des bereits zweifach deprotonierten Puffersubstanz mit Wasser unter Protolyse angibt.

In einer bevorzugten Ausgestaltung der Erfindung kann vorgesehen sein, dass die erste Puffersubstanz einen Siedepunkt und/oder Zersetzungspunkt in einem Bereich aufweist von größer oder gleich 80°C bis kleiner oder gleich 450°C, vorzugsweise größer oder gleich 90°C bis kleiner oder gleich 400°C, besonders bevorzugt von größer oder gleich 100°C bis kleiner oder gleich 300°C, insbesondere von größer oder gleich 110°C bis kleiner oder gleich 160°C.

Dadurch kann vorteilhafter Weise erreicht werden, dass sich der Hilfsstoff auch besonders gut mit einer Verdampfungszusammensetzung auf Basis der Basisflüssigkeit verdampfen lässt. Insbesondere kann dadurch erreicht werden, dass die Konzentration des Hilfsstoffes im Dampf der Verdampfungszusammensetzung sich nur unwesentlich von der Konzentration des Hilfsstoffes in der unverdampften Verdampfungszusammensetzung unterscheidet. Beispielsweise kann sich die Konzentration um weniger als 10%, bevorzugt weniger als 5%, insbesondere weniger als 1% unterscheiden.

In einer bevorzugten Ausgestaltung der Erfindung kann vorgesehen sein, dass die erste Puffersubstanz eine molare Masse aufweist in einem Bereich von größer oder gleich 40 g/mol bis kleiner oder gleich 320 g/mol, vorzugsweise in einem Bereich von größer oder gleich 60 g/mol bis kleiner oder gleich 150 g/mol.

Dadurch kann vorteilhafter Weise erreicht werden, dass der Hilfsstoff eine Resorption etwaigen Nikotins unterstützt.

In einer bevorzugten Ausgestaltung der Erfindung kann vorgesehen sein, dass der Taupunkt der Basisflüssigkeit weniger als 10°C größer als der Siedepunkt der Basisflüssigkeit ist, vorzugsweise weniger als 5°C größer, insbesondere weniger als 1°C größer.

Dadurch kann vorteilhafter Weise erreicht werden, dass die Konzentration des Hilfsstoffes und im Dampf einer Verdampfungszusammensetzung auf Basis der Basisflüssigkeit sich nur unwesentlich von der Konzentration des Hilfsstoffes in der unverdampften Verdampfungszusammensetzung unterscheidet. Beispielsweise kann sich die Konzentration um weniger als 10%, bevorzugt weniger als 5%, insbesondere weniger als 1% unterscheiden.

In einer bevorzugten Ausgestaltung der Erfindung kann vorgesehen sein, dass die erste Puffersubstanz ausgewählt ist aus der Gruppe bestehend aus Carbonsäuren, Sulfonsäuren, Schwefelsäureestern, Phosphonsäuren, Phosphorsäureestern, CH-aciden Verbindungen, NH-aciden Verbindungen, Enolen, Phenolen, oder Mischungen davon.

Es konnte in überraschender Weise gezeigt werden, dass sich diese Stoffe besonders gut als Hilfsstoffe eignen.

In einer bevorzugten Ausgestaltung der Erfindung kann vorgesehen sein, dass die erste Puffersubstanz eine Carbonsäure der folgenden Formel ist:

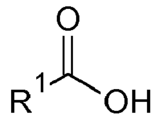

wobei $R^1$ H, -OH, -COOH, C1-8-Alkyl, C2-8-Alkenyl, C1-8-Ketoalkyl, C1-8-Aminoalkyl, Pyridyl, Phenyl, Hydroxyphenyl, Furan, C1-8-Carboxyalkyl, C2-8-Carboxyalkenyl, C1-8-Ketocarboxyalkyl, C1-8-Aminocarboxalkyl, C1-8-Hydroxycarboxyalkyl, C1-8-Dicarboxyalkyl, C1-8-Hydroxydicarboxyalkyl, N-Neuraminsäure oder zyklisches Hydroxymethyltriterpenoidyl ist.

Es konnte in überraschender Weise gezeigt werden, dass sich diese Stoffe besonders gut als die erste Puffersubstanz eignen. Insbesondere weisen diese Stoffe in vorteilhafter Weise eine gut abschätzbare Reaktivität auf. Dadurch kann eine Lagerstabilität gut eingeschätzt werden.

In einer bevorzugten Ausgestaltung der Erfindung kann vorgesehen sein, dass $R^1$ C2-5-Alkyl, C2-3-Alkenyl oder C1-3-alpha-Ketoalkyl ist.

In einer bevorzugten Ausgestaltung der Erfindung kann vorgesehen sein, dass $R^1$ C3-4-Carboxyalkyl, C2-3-Carboxyalkenyl, C1-3-alpha-Ketocarboxyalkyl, C2-3-Aminocarboxalkyl, C3-5-Hydroxycarboxyalkyl, C3-5-Dicarboxyalkyl oder C3-5-Hydroxydicarboxyalky ist.

In einer bevorzugten Ausgestaltung der Erfindung kann vorgesehen sein, dass die erste Puffersubstanz eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin, Benzoesäure, Salicylsäure, Benztraubensäure, Milchsäure, Fumarsäure, Oxalessigsäure, Bernsteinsäure, Buttersäure, Lävulinsäure, Citronensäure, Gluconsäure, Oxalsäure, Malonsäure, Acetessigsäure, Apfelsäure, Weinsäure Ethylendiamintetraessigsäure (EDTA) und Mischungen davon.

In einer besonders bevorzugten Ausgestaltung der Erfindung kann vorgesehen sein, dass der zumindest eine Hilfsstoff eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin, Benzoesäure, Salicylsäure und Mischungen davon.

In einer weiter bevorzugten Ausgestaltung der Erfindung kann vorgesehen sein, dass der zumindest ein Hilfsstoff eine Carbonsäure ist, ausgewählt aus der Gruppe bestehend aus Glyoxylsäure, Propansäure, 2-Oxoglutarsäure, Acrylsäure und Mischungen davon.

In einer bevorzugten Ausgestaltung der Erfindung kann vorgesehen sein, dass die erste Puffersubstanz eine Sulfonsäure der folgenden Formel ist:

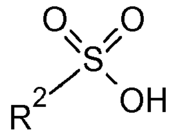

wobei $R^2$ C1-8-Alkyl, C2-8-Alkenyl, oder C1-8-Aminoalkyl ist.

Es konnte in überraschender Weise gezeigt werden, dass sich diese Stoffe besonders gut als die erste Puffersubstanz eignen.

In einer bevorzugten Ausgestaltung der Erfindung kann vorgesehen sein, dass die erste Puffersubstanz eine Sulfonsäure ist, ausgewählt aus der Gruppe bestehend aus Ethansulfonsäure, Methansulfonsäure, Taurin, und Mischungen davon.

In einer bevorzugten Ausgestaltung der Erfindung kann vorgesehen sein, dass die erste Puffersubstanz ein Enol ist, ausgewählt aus der Gruppe bestehend aus Barbitursäure, Ascorbinsäure, und Mischungen davon.

Dadurch kann vorteilhafter Weise erreicht werden, dass mit der Zeit keine unerwünschten Veresterungsreaktionen in der Basisflüssigkeit vorkommen. Zudem kann dadurch insbesondere die Lagerstabilität verbessert werden. Zudem kann dadurch auch die eine Verdampfungszusammensetzung auf Basis einer erfindungsgemäßen Basisflüssigkeit vor unerwünschter Oxidation geschützt werden. Dadurch können insbesondere auch zugesetzte Aromen und/oder zugesetztes Nikotin geschützt werden.

Gemäß einer weiteren Ausgestaltung der Erfindung kann es vorgesehen sein, dass eine erste Puffersubstanz eine Ethylendiamintetraessigsäure ist und zweite Puffersubstanz ein Tris(hydroxymethyl)-aminomethan (TRIS) ist. Hierdurch kann dem sich bildenden Puffersystem neben der Pufferung des pH-Wertes eine Komplexbildner-Funktion zukommen, in welcher es als Chelator zweiwertige Kationen komplexiert.

In einer bevorzugten Ausgestaltung der Erfindung kann vorgesehen sein, dass die Basisflüssigkeit zusätzlich Nikotin aufweist, vorzugsweise in einer Menge in einem Bereich von größer oder gleich 1 mg/mL bis kleiner oder gleich 100 mg/mL bezogen auf die Basisflüssigkeit aufweist, besonders bevorzugt in einem Bereich von größer oder gleich 2 mg/mL bis kleiner oder gleich 20 mg/mL, beispielsweise in einem Bereich von

größer oder gleich 4 mg/mL bis kleiner oder gleich 10 mg/mL.

Dadurch kann vorteilhafter Weise eine Nikotinkonzentration erreicht werden, durch die beim Konsum das Nikotin eine ausreichende physiologische Wirkung erzielt, ohne Rachen und Lungen übermäßig zu reizen.

In einer Ausgestaltung der Erfindung kann vorgesehen sein, dass die Pufferkapazität des Puffers so eingestellt ist, dass sich der pH-Wert durch die Zugabe des Nikotins um weniger als 1 ändert.

In einer bevorzugten Ausgestaltung der Erfindung kann vorgesehen sein, dass die Basisflüssigkeit zusätzlich Aroma aufweist, vorzugsweise in einer Menge in einem Bereich von größer oder gleich 0,1 gew.-% bis kleiner oder gleich 10 gew.-% bezogen auf die Basisflüssigkeit, besonders bevorzugt in einem Bereich von größer oder gleich 0,5 gew.-% bis kleiner oder gleich 5 gew.-%, beispielsweise in einem Bereich von größer oder gleich 1 gew.-% bis kleiner oder gleich 1 gew.-%.

Dadurch kann vorteilhafter Weise erreicht werden, dass die Verdampfungszusammensetzung einen angenehmen Geschmack und/oder Geruch aufweist. Insbesondere kann durch die erfindungsgemäße Verdampfungszusammensetzung vorteilhafter weise erreicht werden, dass etwaiges Nikotin das Aroma nicht negativ beeinträchtigt. Durch die Basisflüssigkeit kann zudem erreicht werden, dass das Aroma stabil ist.

In einer Ausgestaltung der Erfindung kann vorgesehen sein, dass die Pufferkapazität des Puffers so eingestellt ist, dass sich der pH-Wert durch die Zugabe des Aromas um weniger als 1 ändert.

In einer bevorzugten Ausgestaltung der Erfindung kann vorgesehen sein, dass die Basisflüssigkeit eine Zusammensetzung aufweist bestehend aus Propylenglykol in einem Bereich von größer oder gleich 0 gew.-% bis kleiner oder gleich 90 gew.-%, Glycerin in einem Bereich von größer oder gleich 0 gew.-% bis kleiner oder gleich 90 gew.-% und Wasser in einem Bereich von größer oder gleich 0 gew.-% bis kleiner oder gleich 20 gew.-%.

Beispielsweise kann die Verdampfungszusammensetzung vorteilhafter Weise ein Massenverhältnis von Propylenglykol zu Glycerin zu Wasser aufweisen von 55:35:10, alternativ von 50:50:0, weiter alternativ von 25:75:0, oder von 30:70:0, 0:80:20, oder auch 90:0:10.

## Patentansprüche

1. Basisflüssigkeit für Verdampfungszusammensetzungen zum Inhalieren, bestehend zumindest aus:

   - einem Hilfsstoff,
   - und als Rest einer Mischung bestehend aus Propylenglykol in einem Bereich von größer oder gleich 0 gew.-% bis kleiner oder gleich 100 gew.-%, Glycerin in einem Bereich von größer oder gleich 0 gew.-% bis kleiner oder gleich 100 gew.-% und Wasser in einem Bereich von größer oder gleich 0 gew.-% bis kleiner oder gleich 50 gew.-%,

   wobei der Hilfsstoff einen Puffer bildet und zumindest aus einer ersten und einer zweiten Puffersubstanz eines Puffersystems besteht.

2. Basisflüssigkeit für Verdampfungszusammensetzungen zum Inhalieren nach Anspruch 1, wobei die zweite Puffersubstanz eine korrespondierende Base oder eine korrespondierende Säure der ersten Puffersubstanz ist.

3. Basisflüssigkeit für Verdampfungszusammensetzungen zum Inhalieren nach einem der vorherigen Ansprüche, wobei der Hilfsstoff ein molares Verhältnis von der ersten Puffersubstanz zu der zweiten Puffersubstanz aufweist in einem Bereich von größer oder gleich 0,25:1 bis kleiner oder gleich 4: 1, vorzugsweise in einem Bereich von größer oder gleich 0,5:1 bis kleiner oder gleich 2:1, besonders bevorzugt in einem Bereich von größer oder gleich 0,9:1 bis kleiner oder gleich 1,1:1

4. Basisflüssigkeit für Verdampfungszusammensetzungen zum Inhalieren nach einem der vorherigen Ansprüche, wobei die zweite Puffersubstanz ein Salz ist, vorzugsweise ausgewählt aus der Gruppe bestehend aus Ammonium-, Lithium-, Natrium-, Kalium-, Magnesium- und/oder Calcium-Salzen oder aus der Gruppe bestehend aus Chlorid-, Bromid-, Iodid-, Sulfat-, Phosphat-, Nitrat-, Carbonat- und Thiocyanat-Salzen.

5. Basisflüssigkeit für Verdampfungszusammensetzungen zum Inhalieren nach einem der vorherigen Ansprüche, wobei die Basisflüssigkeit den zumindest einen Hilfsstoff in einem Verhältnis bezogen auf die Basisflüssigkeit aufweist in einem Bereich von größer oder gleich 0,2 gew.-% bis kleiner oder gleich 20 gew.-%, vorzugsweise von größer oder gleich 0,5 gew.-% bis kleiner oder gleich 10 gew.-%, besonders bevorzugt von größer oder gleich 1 gew.-% bis kleiner oder gleich 5 gew.-%

6. Basisflüssigkeit für Verdampfungszusammensetzungen zum Inhalieren nach Anspruch 1, wobei die erste Puffersubstanz einen ersten pKs-Wert aufweist in einem Bereich von größer oder gleich -5 bis kleiner oder gleich 10, vorzugsweise größer oder gleich -2 bis kleiner oder gleich 5, besonders bevorzugt größer oder gleich -2 bis kleiner oder gleich 4, und optional einen zweiten und weiter optional dritten pKs-Wert aufweist in einem Bereich von größer oder gleich 0 bis kleiner oder gleich 10, vorzugsweise größer oder gleich 2 bis kleiner oder gleich 8, besonders bevorzugt größer oder gleich 3,5 bis kleiner oder gleich 6,5.

**7.** Basisflüssigkeit für Verdampfungszusammensetzungen zum Inhalieren nach einem der vorherigen Ansprüche, wobei die erste Puffersubstanz einen Siedepunkt und/oder Zersetzungspunkt in einem Bereich aufweist von größer oder gleich 80°C bis kleiner oder gleich 450°C, vorzugsweise größer oder gleich 90°C bis kleiner oder gleich 400°C, besonders bevorzugt von größer oder gleich 100°C bis kleiner oder gleich 300°C, insbesondere von größer oder gleich 110°C bis kleiner oder gleich 160°C.

**8.** Basisflüssigkeit für Verdampfungszusammensetzungen zum Inhalieren nach einem der vorherigen Ansprüche, wobei die erste Puffersubstanz ausgewählt ist aus der Gruppe bestehend aus Carbonsäuren, Sulfonsäuren, Schwefelsäureestern, Phosphonsäuren, Phosphorsäureestern, CH-aciden Verbindungen, NH-aciden Verbindungen, Enolen, Phenolen, oder Mischungen davon.

**9.** Basisflüssigkeit für Verdampfungszusammensetzungen zum Inhalieren nach einem der vorherigen Ansprüche, wobei die erste Puffersubstanz eine Carbonsäure der folgenden Formel ist:

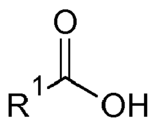

wobei $R^1$ -H, -OH, -COOH, C1-8-Alkyl, C2-8-Alkenyl, C1-8-Ketoalkyl, C1-8-Aminoalkyl, Pyridyl, Phenyl, Hydroxyphenyl, Furan, C1-8-Carboxyalkyl, C2-8-Carboxyalkenyl, C1-8-Ketocarboxyalkyl, C1-8-Aminocarboxalkyl, C1-8-Hydroxycarboxyalkyl, C1-8-Dicarboxyalkyl, C1-8-Hydroxydicarboxyalkyl, N-Neuraminsäure oder zyklisches Hydroxymethyltriterpenoidyl ist.

**10.** Basisflüssigkeit für Verdampfungszusammensetzungen zum Inhalieren nach einem der vorherigen Ansprüche, wobei die erste Puffersubstanz eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin, Benzoesäure, Salicylsäure, Ethylentriamintetraessigsäure (EDTA) und Mischungen davon.

**11.** Basisflüssigkeit für Verdampfungszusammensetzungen zum Inhalieren nach einem der vorherigen Ansprüche, wobei die erste Puffersubstanz eine Sulfonsäure der folgenden Formel ist:

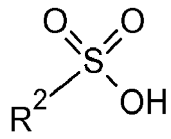

wobei $R^2$ C1-8-Alkyl, C2-8-Alkenyl, oder C1-8-Aminoalkyl ist.

**12.** Basisflüssigkeit für Verdampfungszusammensetzungen zum Inhalieren nach einem der vorherigen Ansprüche, wobei die erste Puffersubstanz eine Sulfonsäure ist, ausgewählt aus der Gruppe bestehend aus Ethansulfonsäure, Methansulfonsäure, Taurin, und Mischungen davon.

**13.** Basisflüssigkeit für Verdampfungszusammensetzungen zum Inhalieren nach einem der vorherigen Ansprüche, wobei die erste Puffersubstanz ein Enol ist, ausgewählt aus der Gruppe bestehend aus Barbitursäure, Ascorbinsäure, und Mischungen davon.

**14.** Basisflüssigkeit für Verdampfungszusammensetzungen zum Inhalieren nach einem der vorherigen Ansprüche, wobei die Basisflüssigkeit zusätzlich Aroma aufweist, vorzugsweise in einer Menge in einem Bereich von größer oder gleich 0,1 gew.-% bis kleiner oder gleich 10 gew.-% bezogen auf die Basisflüssigkeit, besonders bevorzugt in einem Bereich von größer oder gleich 0,5 gew.-% bis kleiner oder gleich 5 gew.-%, beispielsweise in einem Bereich von größer oder gleich 1 gew.-% bis kleiner oder gleich 1 gew.-%.

**15.** Basisflüssigkeit für Verdampfungszusammensetzungen zum Inhalieren nach einem der vorherigen Ansprüche, wobei die Basisflüssigkeit zusätzlich Nikotin aufweist, vorzugsweise in einer Menge in einem Bereich von größer oder gleich 1 mg/mL bis kleiner oder gleich 100 mg/mL bezogen auf die Basisflüssigkeit aufweist, besonders bevorzugt in einem Bereich von größer oder gleich 2 mg/mL bis kleiner oder gleich 20 mg/mL, beispielsweise in einem Bereich von größer oder gleich 4 mg/mL bis kleiner oder gleich 10 mg/mL.

Europäisches Patentamt
European Patent Office
Office européen des brevets

## EUROPÄISCHER RECHERCHENBERICHT

Nummer der Anmeldung
EP 19 16 6142

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2015/374035 A1 (SANCHEZ LUIS A [US] ET AL) 31. Dezember 2015 (2015-12-31)<br>* Absätze [0002], [0004], [0008]; Tabellen I-III * | 1-13<br>14,15 | INV.<br>A61K9/00<br>A61K47/12<br>A61K47/20<br>A61K47/10<br>A61K31/465<br>A24D1/00 |
| Y | WO 2019/005889 A1 (NUDE NICOTINE INC [US]; RUBENSTEIN JACOB [US])<br>3. Januar 2019 (2019-01-03)<br>* Seite 42 - Seite 43 * | 1-15 | |
| Y | US 2017/334881 A1 (DULL GARY M [US] ET AL)<br>23. November 2017 (2017-11-23)<br>* Absatz [0132]; Anspruch 8 * | 1-15 | |
| Y | US 2017/333415 A1 (WILLIAMS JONNIE R [US])<br>23. November 2017 (2017-11-23)<br>* Absätze [0022], [0081] * | 1-15 | |
| | | | RECHERCHIERTE SACHGEBIETE (IPC) |
| | | | A61K<br>A24F<br>A24D |

Der vorliegende Recherchenbericht wurde für alle Patentansprüche erstellt

1

| Recherchenort | Abschlußdatum der Recherche | Prüfer |
|---|---|---|
| München | 4. Oktober 2019 | Wörth, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 19 16 6142

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-10-2019

| Im Recherchenbericht angeführtes Patentdokument | Datum der Veröffentlichung | Mitglied(er) der Patentfamilie | Datum der Veröffentlichung |
|---|---|---|---|
| US 2015374035 A1 | 31-12-2015 | CN 104968225 A | 07-10-2015 |
| | | EP 2948006 A1 | 02-12-2015 |
| | | GB 2513061 A | 15-10-2014 |
| | | HK 1203128 A1 | 23-10-2015 |
| | | US 2015374035 A1 | 31-12-2015 |
| | | WO 2014116974 A1 | 31-07-2014 |
| WO 2019005889 A1 | 03-01-2019 | KEINE | |
| US 2017334881 A1 | 23-11-2017 | CN 106536501 A | 22-03-2017 |
| | | EP 3148982 A1 | 05-04-2017 |
| | | JP 2017523133 A | 17-08-2017 |
| | | US 2015344456 A1 | 03-12-2015 |
| | | US 2017334881 A1 | 23-11-2017 |
| | | WO 2015183801 A1 | 03-12-2015 |
| US 2017333415 A1 | 23-11-2017 | US 2017333415 A1 | 23-11-2017 |
| | | WO 2016133890 A1 | 25-08-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **B. KOSZOWSKI ; L. C. VIRAY ; S. B. STANFILL ; J. G. LISKOB ; Z. R. ROSENBERRY ; J. L. POTTS ; W. B. PICKWORTH.** *Pharmacol Biochem Behav.,* 2015, vol. 136, 1-6
- **R. V FANT ; J. E HENNINGFIELD ; R. A NELSON ; W. B PICKWORTH.** *Tobacco Control,* 1999, vol. 8, 387-392
- **W. B. PICKWORTH ; Z. R. ROSENBERRY ; W. GOLD ; B. KOSZOWSKI.** *J Addict Res Ther,* 2014, vol. 5 (3), 1